# EUROPEAN PATENT APPLICATION

(11) **EP 1 627 593 A1**
(43) Date of publication of application: **22.02.2006**
(21) Application number: 04715519.7
(22) Date of filing: 27.02.2004
(51) Int. Cl.: A61B 1/00

(54) **MEDICAL IMAGE RECORDER, METHOD FOR DISPLAYING ENDOSCOPE IMAGE, METHOD FOR TAKING IN ENDOSCOPE IMAGE, AND PORTABLE STORAGE MEDIUM**

(30) Priority: 27.05.2003 JP 2003149571; 25.12.2003 JP 2003430428
(71) Applicant: Olympus Corporation, Tokyo 151-0072 (JP)
(72) Inventor: SHOJI, Hideyuki, Shibuya-ku Tokyo 1510072 (JP); OHSHIMA, Mutsumi, Shibuya-ku Tokyo 1510072 (JP); MIYOSHI, Yoshitaka, Shibuya-ku Tokyo 1510072 (JP); ODA, Tomohiko, Shibuya-ku Tokyo 1510072 (JP); ITO, Nobuyasu, Shibuya-ku Tokyo 1510072 (JP); ETO, Tadao, Shibuya-ku Tokyo 1510072 (JP); IJICHI, Toshiro, Shibuya-ku Tokyo 1510072 (JP); ISHIBASHI, Katsuyoshi, Shibuya-ku Tokyo 1510072 (JP); KIKKAWA, Masashi; C/o Olympus Systems Corporation, Shibuya-ku Tokyo, 1510061 (JP); NAKATSUCHI, Kazutaka, Shibuya-ku Tokyo 1510072 (JP)
(74) Representative: von Hellfeld, Axel
(86) International application number: PCT/JP2004/002433
(87) International publication number: WO 2004/105591

(57) **Abstract**

A medical image recording apparatus records an endoscopic image captured by an endoscope, and includes a generation unit for generating image data used for display data on a monitor, and a composition unit for combining the endoscopic image captured by the endoscope with the image data, and outputting the composition result to the monitor.

## Description

### Technical Field

The present invention relates to a medical image recording apparatus for recording an endoscopic image captured by an endoscope.

### Background Art

Recently, an endoscopic examination has been generally used for directly observing the body cavity and the lumen of entrails by inserting the thin and long end unit of a scope into a target portion in a target portion to be examined such as a coelom, etc.

In addition, an endoscopic image filing system has also been widely used in recording and accumulating an endoscopic image captured by an endoscopic apparatus in a server connected over a network, and retrieving the image as necessary.

In the endoscopic image filing system, an operator such as an endoscopist, etc. presses a switch, for example, a release switch provided for a scope portion of the endoscopic apparatus so that the medical image recording apparatus connected to the endoscopic apparatus can take an endoscopic image displayed on the monitor, and the information about a patient and the information about examination conditions, etc. can be added and recorded in the server over the network.

For example, in the endoscopic image filing system disclosed by the patent literature 1, an endoscopic image captured by the endoscopic observation apparatus is processed by compressing the image data by the medical image recording apparatus, transmitted to a file server over a network, and recorded and accumulated in the server.

### Patent Literature 1

Japanese Patent Application Laid-open No. Hei 7-141498

An endoscopic image fetched in the endoscopic examination is used later in preparing a report and an electronic chart. At this time, the image displaying the portion to be examined and its position are located only based on the memory of the operator. For example, when a sample of tissue is removed for a pathological examination, etc. (hereinafter referred to as a biopsy), and when a report is prepared after the endoscopic examination, the position of the biopsy is designated based on the memory of the operator while watching the image displayed on the screen of a terminal or printed on a printer, and the designated position is marked on the endoscopic image to be attached to the report. Therefore, the report preparing operation is complicated and requires a long time.

### Disclosure of Invention

The present invention aims at providing a medical image recording apparatus capable of reducing the time and labor required in later selecting an image and designating the necessary position in the image.

Moreover the present invention aims at providing a medical image recording apparatus capable of adding information while checking information on the monitor, when an operator adds information to a fetched endoscopic image.

The medical image recording apparatus according to the first embodiment according to the present invention is based on recording an endoscopic image capture by an endoscope to solve the above-mentioned problem, and includes a generation unit and a composition unit.

The generation unit generates image data for displaying an image on a monitor at an instruction of an operator.

The composition unit combines the endoscopic image capture by the endoscope with the image data, and outputs the result to the monitor.

With the above-mentioned configuration, an instruction of an operator is displayed on the monitor together with an endoscopic image. Therefore, the operator can input data while checking his or her instruction on the monitor.

Furthermore, if the generation of the image data is stopped when a predetermined time passes after the generation unit generates the image data, then the displayed input instruction of the operator does not interfere with the endoscopic image.

In addition, the medical image recording apparatus according to the second embodiment of the present invention includes a generation unit and a recording unit.

The generation unit generates image data for displaying an image on a monitor at an instruction of an operator.

The recording unit records the image data after associating the information based on the endoscopic image captured by the endoscope with the information based on the image data.

With this configuration, the instruction of the operator is stored as associated with an endoscopic image. Therefore, when the endoscopic image is displayed later, the instruction of the operator can also be displayed together.

### Brief Description of Drawings

FIG. 1 shows the first example of the configuration of the endoscopic image filing system according to an embodiment of the present invention;
FIG. 2 shows the second example of the configuration of the endoscopic image filing system according to an embodiment of the present invention;
FIG. 3 shows an example of the configuration of the image recording apparatus;
FIGS. 4A, 4B, and 4C show examples of a display on a monitor according to the first embodiment of the present invention;
FIGS. 5A and 5B show examples of a display on a monitor according to the second embodiment of the present invention; and
FIG. 6 is a flowchart showing the process of operating the image recording apparatus.

### Best Mode for Carrying out the Invention

Described below is an embodiment of the present invention.

In the endoscopic image filing system according to an embodiment of the present invention, an instruction is issued during the endoscopic examination or after the examination on the monitor screen which displays a captured image. Therefore, various information can be added to the captured image by applying a mark to the position of the biopsy and the suspicious portion in the examination, and the information can be recorded with the captured endoscopic image. As a result, during the endoscopic examination or after the examination, a mark is added to the captured image displayed on the monitor, and the marked image can be used in later preparing a report, thereby reducing the time required to finish the report.

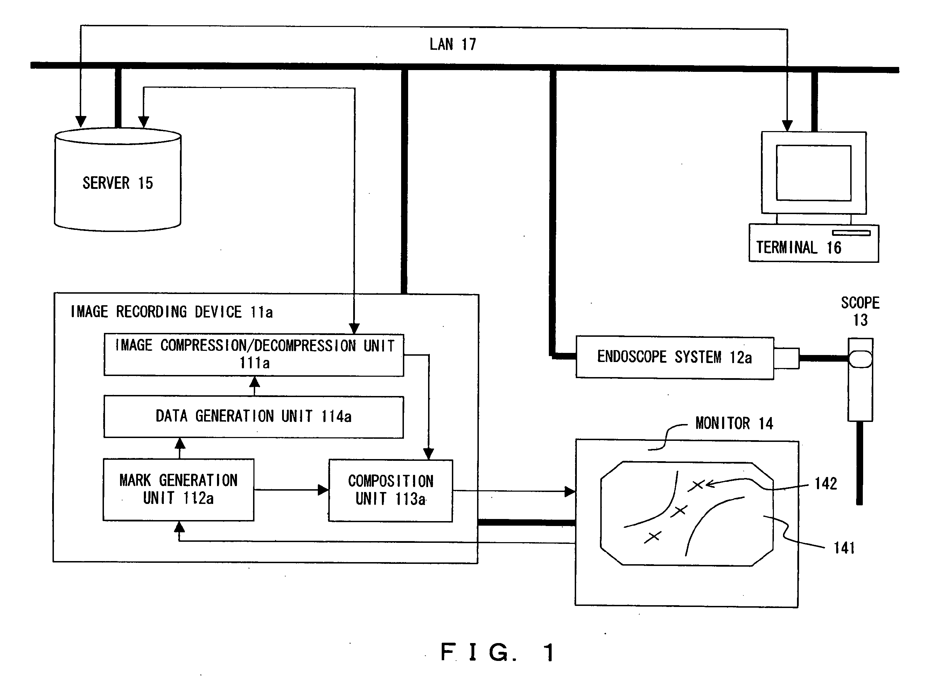

FIG. 1 shows the first example of the configuration of the endoscopic image filing system according to an embodiment of the present invention.

In the system according to the first embodiment, information such as a mark on a captured endoscopic image, etc. is added after the examination to the image captured in the endoscopic examination.

The endoscopic image filing system shown in FIG. 1 comprises an image recording device 11a, an endoscopic system 12a, a scope 13, a monitor 14, a server 15, and a terminal 16.

Among the components, the image recording device 11a, the endoscopic system 12a, the server 15, and the terminal 16 are connected over a network by a LAN 17. Through the LAN 17, an image captured by the endoscopic system 12a is recorded and accumulated in the server 15. When information is added to an endoscopic image, the image recording device 11a reads data such as a captured image, etc. from the server 15. When a report is prepared later, the terminal 16 reads the data. In addition, the monitor 14 is connected to the image recording device 11a through a dedicated line, and the data such as an image read by the image recording device 11a from the server 15, etc. is displayed on the monitor 14.

In this system, a plural units of the image recording devices 11a and terminals 16 can be included. It is also possible in the system to store data not in the server in the network, but in the memory, a hard disk, a DVD drive, from which data is read when a report is prepared, in the image recording apparatus by configuring the system as a non-network system.

The image recording device 11a reads information such as a captured image, etc. stored in the server 15 after the examination and displays it on the monitor 14, and generates an image obtained by processing the captured image according to the instruction input from the operator.

The endoscopic system 12a comprises a luminous source apparatus, not shown in the attached drawings, for emitting illuminating light for illuminating a target portion through the scope 13, an image processing apparatus for performing various signal processes using an observation image of the target portion obtained by the illumination from the luminous source apparatus as a picture signal, and an observation monitor for display of the picture signal. The endoscopic system 12a converts the observation image of the target portion captured using the scope 13 into a picture signal which is stored as a captured image in the server 15 through the LAN 17.

The scope 13 performs a capturing operation, the end unit of the scope 13 is inserted into a patient to be examined, the light from the luminous source apparatus is reflected and is opto-electrically converted by a solid-state image pickup device such as a CCD, etc., and output to the endoscopic system 12a. The scope 13 is also provided with a scope switch, which has the function of the release switch, etc. for an instruction to capture an image, not shown in the attached drawings, but mounted in the position where the operator can handle it while proceeding with the examination.

The monitor 14 is a display monitor for displaying an image read from the server 15 when the image recording device 11a processes an image. The display unit of the monitor 14 is a touch screen, and an input instruction can be issued by designation with a finger, etc. on the screen. When the operator inputs an instruction by touching the screen by the finger, etc. over an endoscopic image 141, a mark 142 is displayed on the endoscopic image 141 displayed on the monitor 14.

The server 15 is a file server storing as a database the personal information about a patient obtained by an endoscope, a captured image, etc. The terminal 16 is an information processing device connected to the server 15 over a network by the LAN 17. A doctor who has made an endoscopic examination generates an electronic chart and an examination report while calling the endoscopic image and patient information fetched from the server 15 using the terminal 16.

Furthermore, the image recording device 11a shown in FIG. 1 comprises an image compression/decompression unit 111a, a mark generation unit 112a, a composition unit 113a, and a data generation unit 114a.

The image compression/decompression unit 111a decompresses the data of an endoscopic image read from the server 15, and compresses data generated by the data generation unit 114a. The mark generation unit 112a converts a mark, etc. input by the operator on the display screen of the monitor 14 into display data. The composition unit 113a combines the display data generated by the mark generation unit 112a with the endoscopic image read from the server 15 to generate display screen data, and outputs the resultant data to the monitor 14. When the operator issues an instruction to store an image, the data generation unit 114a generates data to be stored in the server 15 based on the image displayed on the monitor 14 according to the data read from the server 15 and the information about the display data obtained from the mark generation unit 112a.

The image recording device 11a displays the image data of the endoscopic image from the server 15 on the monitor 14, and combines and displays the information such as a mark, etc. on the display screen at an operation instruction of the operator on the display screen of the monitor 14. At an instruction of the operator, the data is generated as associated with the endoscopic image displayed with the added information such as a mark, etc. The generated data is compressed by the image compression unit 111a, transmitted to the server 15, and entered in the database of the server 15.

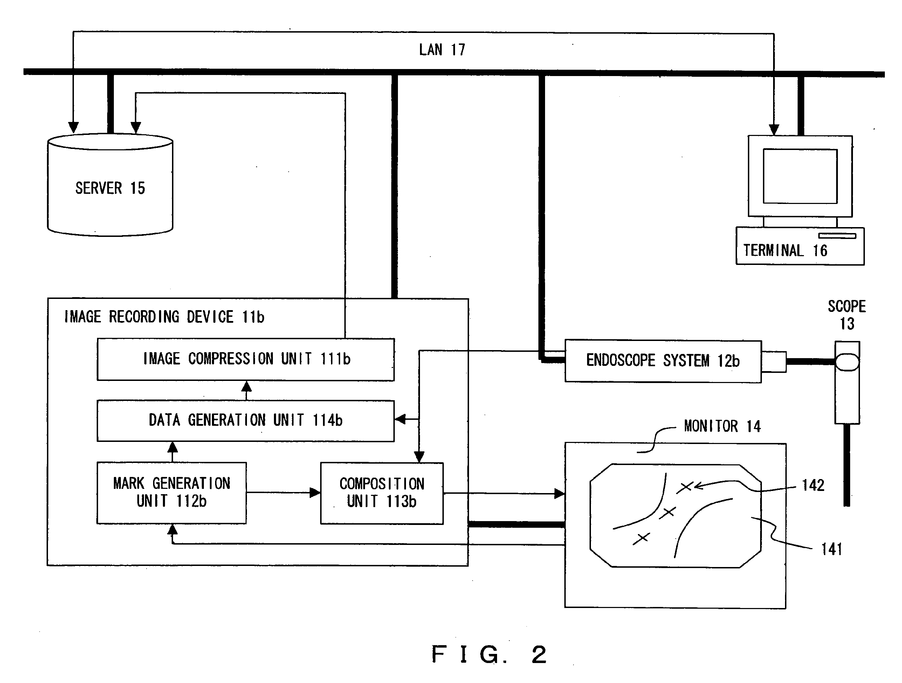

FIG. 2 shows the second example of the configuration of the endoscopic image filing system according to an embodiment of the present invention.

The system of the second example of the configuration can add various information to a captured image while performing an examination by marking the position of the biopsy and a suspicious portion in the examination by an instruction of the operator over the display screen on the monitor 14 while performing the endoscopic examination, and the information can be recorded with the captured endoscopic image.

In the configuration shown in FIG. 2, an image recording device 11b and an endoscopic system 12b are different from the corresponding components in the configuration shown in FIG. 1. The endoscopic system 12b is directly connected to the image recording device 11b through a dedicated line. The data of the observation image is transmitted from the endoscopic system 12b directly to the image recording device 11b, and displayed on the monitor 14 connected to the image recording device 11b. The image captured by the endoscopic system 12b is stored in the server 15 through the image recording device 11b. At this time, if the operator issues an instruction to process an image being captured by the image recording device 11b by, for example, adding the mark 142, etc. to the displayed image while watching the display screen 141 on the monitor 14, then the information such as the mark 142, etc. can be combined with the captured image, displayed on the monitor 14, and can be stored in the server 15 together with the captured image as added information.

The image recording device 11b comprises an image compression unit 111b instead of the image compression/decompression unit 111a. The image compression unit 111b compresses data generated by a data generation unit 114b.

In the image recording device 11b, a composition unit 113b combines the image data of the endoscopic image transmitted from the endoscopic system 12b with the information such as a mark, etc. generated by a mark generation unit 112b at an operation instruction of the operator on the display screen of the monitor 14, and displays the composite data on the monitor 14. When the endoscopic image is fetched by the operator pressing the release switch, the data generation unit 114b generates the data with the information such as the mark, etc. associated with the endoscopic image displayed on the screen, compresses the data by the image compression unit 111b, transmits the result to the server 15, and enters it in the database of the server 15.

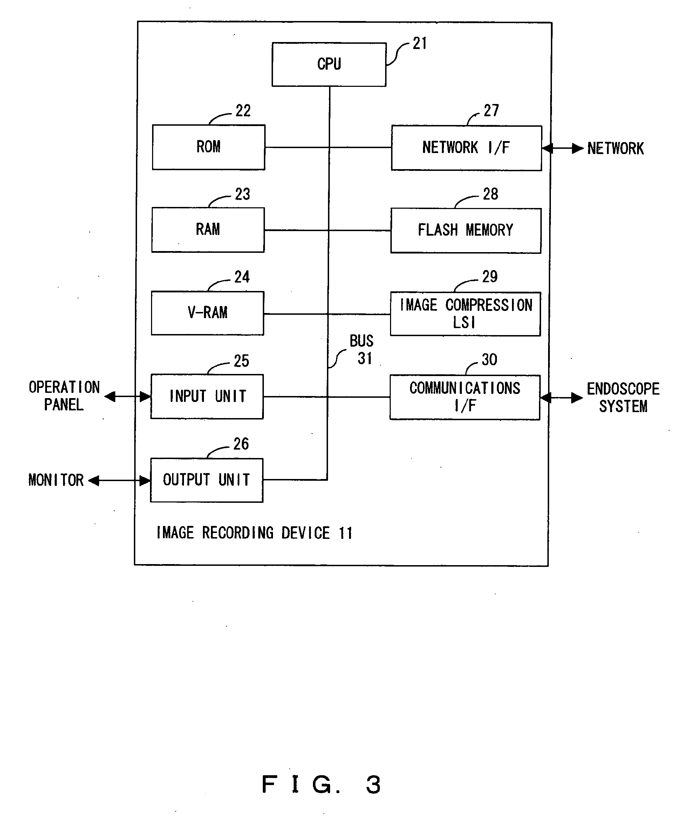

FIG. 3 shows an example of the configuration of the image recording devices 11a and 11b.

The image recording devices 11a and 11b shown in FIG. 3 comprise a CPU 21 for controlling various operations, ROM 22 for storing a control program, etc., RAM 23 which functions as working memory during an operation, video RAM (V-RAM) 24 for temporarily storing an image signal from an endoscopic system 12, an input unit 25 for receiving input from a pointing device, an operation panel, an input device of a keyboard, etc., a touch screen of the monitor 14, and a reading device of a portable storage medium such as CD-ROM, etc., an output unit 26 for output of image data, etc. to the monitor 14, a network interface (network I/F) 27 for communicating a signal such as data, an instruction, etc. with a network, nonvolatile flash memory 28 for storing compressed image data to be transmitted to the server 15, an image compression LSI 29 for compressing an image signal, and a communications interface (communications I/F) 30 (only for the image recording device 11b) for communicating a communications signal with the endoscopic systems 12 (12a and 12b). These components are connected through a bus line 31.

In the image recording devices 11a and 11b shown in FIG. 3, the CPU 21 executes the firmware in the ROM 22 or the program read by the input unit 25 from a portable storage medium to control other components, performs a process for display on the monitor 14, a mark displaying process, and various processes described in the present specifications such as the processes for data storage in the server 15, etc. The image recording devices 11a and 11b are not only configured to realize a process like software by the CPU 21 shown in FIG. 3 executing a program, but can be configured to realize a process entirely or partially by hardware.

Described below is the operation performed during the endoscopic examination in the system according to the first example of the configuration shown in FIG. 1.

In the first example of the configuration, if the operator operates the scope 13 while watching the endoscopic image displayed on the observation monitor provided in the endoscopic system 12a but not shown in the attached drawings, presses the release switch provided for the scope 13 as necessary, and fetches an endoscopic image as a still image, then the data of the endoscopic image is compressed, transmitted to the server 15 through the LAN 17, and entered in the database.

After the examination, the operator reads the data of the endoscopic image entered in the server 15 to the image recording device 11a, and the data is decompressed by the image compression/decompression unit 111a and displayed on the monitor 14. While watching the screen, the operator specifies the position to be marked with the mark 142 using the finger, etc. over the endoscopic image 141 on the monitor 14 for the portion such as the position of the biopsy, the suspicious portion detected on the monitor, etc. The position is announced to the image recording device 11a as coordinate data on the screen of the monitor 14. Upon receipt of the data, the mark generation unit 112a of the image recording device 11a generates the display data for display of a mark such as "x", etc. in the position specified by the operator, outputs the data to the composition unit 113a, and notifies the data generation unit 114a of the information such as the display position of the mark, the type of mark, etc.

The composition unit 113a combines the display data of the mark from the mark generation unit 112a with the image data from the endoscopic system 12a, and outputs the result to the monitor 14. Thus, the mark such as "x", etc. is displayed in the position specified by the operator for an endoscopic image 141a.

The mark generation unit 112a manages the display time of a display element such as a mark, etc. displayed on the monitor 14 so that the output of the display data of each display element can be stopped when a predetermined time passes from the time of outputting the display data to the composition unit 113a, and the display of the display element can be cleared on the monitor 14. Thus, the mark 142 displayed on the monitor 14 is cleared from the screen when a predetermined time passes after the start of display, thereby preventing the display of the mark 142 from interfering with the endoscopic image 141. According to an example in the present embodiment, the combination and display of the mark 142 is stopped after the passage of a predetermined time. It is further convenient to provide a selection system for selecting whether or not the display or the combination of the mark 142 with the endoscopic image 141 is specified at an selection instruction of an operator so that the operation of the composition unit 113a can be selectively controlled.

When the operator issue a storage instruction for an image after completing the input instruction for the mark 142, etc. on the monitor 14, the data generation unit 114a generates entry data by adding mark information (about the display position, the type, etc.) about the mark notified of by the mark generation unit 112a to the image data of the displayed endoscopic image, compresses the data by the image compression/decompression unit 111a, and transmits the data to the server 15 through the LAN 17 for storage in the server 15.

Thus, the mark 142 is displayed in the position specified by the operator on the display screen of the monitor 14. When the endoscopic image 141 is fetched, the information about the mark 142 is added to the image data of the endoscopic image, and the data is stored and accumulated in the server 15. Therefore, the operator can refer to the endoscopic image with the mark added to the data when the operator refers to the fetched endoscopic image in later preparing a report. As a result, the position of a biopsy, etc. can be easily recognized by the mark. In addition, when the image is used as is, the operator can be free of the laborious operation of newly generating an image with a mark of a biopsy position added thereto, thereby reducing the time required to prepare a report. Since the data of an endoscopic image and the mark information can be stored as separate data in the server 15, the operator can optionally switch the display between the image with a mark and the image only when the endoscopic image stored in the server 15 is displayed later.

Described below is the operation performed when an endoscopic examination is performed by the system according to the second example of the configuration shown in FIG. 2.

When an endoscopic examination is performed, an operator such as an endoscopist, etc. first inputs the examination ID assigned to the endoscopic examination, and the identification information about the patient such as the ID of the patient to be examined (including the personal information about the patient such as the name, age, sex, etc. when the patient has not been entered in the server 15) through the input device such as a keyboard, a card reader, etc. of the endoscopic system 12 provided by the bed of the patient. With the input of the information, the image recording device 11b reads the personal information about the patient from the server 15 to generate the header information to be added to the image data of the fetched endoscopic image.

Then, the operator operates the scope 13 while watching the endoscopic image 141 displayed on the monitor 14, presses the release switch provided for the scope 13 as necessary, and fetches an endoscopic image as a still image. When the release switch is pressed, the image recording device 11b compresses image data input from the endoscopic system 12, and the compressed image data is accompanied with the header information including the personal information about a patient, the capture information about the image, etc., and then transmitted to the server 15 through the LAN 17 for storage in the server 15.

The operator specifies the position to be marked with the mark 142 using the finger, etc. over the endoscopic image 141 on the monitor 14 for the portion such as the position of the biopsy, the suspicious portion detected on the monitor, etc. The position is announced to the image recording device 11b as coordinate data on the screen of the monitor 14. Upon receipt of the data, the mark generation unit 112b of the image recording device 11b generates the display data for display of a mark such as "x", etc. in the position specified by the operator, outputs the data to the composition unit 113b, and notifies the data generation unit 114b of the information such as the display position of the mark, the type of mark, etc.

The composition unit 113b combines the display data of the mark from the mark generation unit 112b with the image data from the endoscopic system 12, and outputs the result to the monitor 14. Thus, the mark such as "x", etc. is displayed in the position specified by the operator for an endoscopic image 141. As with the mark generation unit 112a according to the first example of the configuration, the mark generation unit 112b manages the display time of a display element such as a mark, etc. displayed on the monitor 14 so that the output of the display data can be stopped when a predetermined time passes, and the display of the display element can be cleared on the monitor 14. The image recording device 11b can further comprise a selecting unit for allowing an operator to select and set whether or not the mark 142 is to be combined and displayed with the endoscopic image 141, and selectively controlling the display based on the setting.

When the operator presses the release switch and specifies the fetch of an endoscopic image with the mark 142 displayed on the monitor 14, the data generation unit 114b generates entry data by adding the mark information (about the display position, the type, etc.) about the mark notified of by the mark generation unit 112b to the image data from the endoscopic system 12, compresses the data by the image compression unit 111b, and transmits the resultant data to the server 15 through the LAN 17 for storage in the server 15.

Thus, in the system according to the second example of the configuration, the mark 142 is displayed in the position specified by the operator on the display screen of the monitor 14 on which an observation image is displayed in an endoscopic examination. When the endoscopic image 141 is fetched, the information about the mark 142 is added to the image data of the endoscopic image, and the data is stored and accumulated in the server 15. Therefore, the operator can refer to the endoscopic image with the mark added at the time of the endoscopic examination to the data when the operator refers to the fetched endoscopic image in later preparing a report.

In the system according to the second example of the configuration, since the data of an endoscopic image and the mark information can be stored as separate data in the server 15, the operator can optionally switch the display between the image with a mark and the image only when the endoscopic image stored in the server 15 is displayed later.

Described below is the type of information to be added to an endoscopic image.

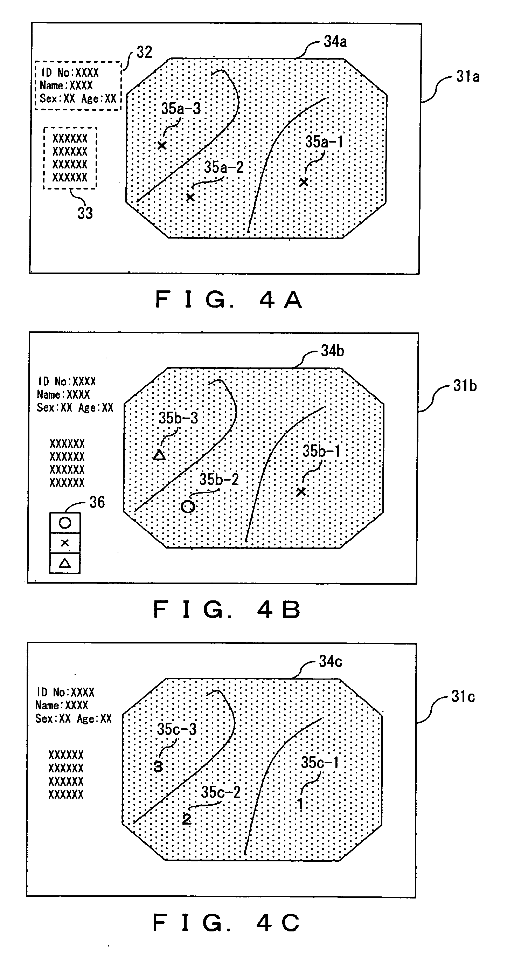

FIG. 4 shows an example of a display of the monitor 14 according to the first embodiment of the present invention.

In the first embodiment, a mark is input using a touch screen of the monitor 14.

In the example of a display shown in FIG. 4A, an endoscopic image 34a next to patient information 32 such as a patient ID, a patient name, etc., and examination condition information 33 such as a machine name used in an examination, etc. is displayed on a display screen 31a of the monitor 14. While watching the endoscopic image 34a, the operator touches the position to be marked on the endoscopic image 34a with the finger, etc. Then, a "x" mark 35a is displayed in the position. FIG. 4A shows three marked positions with marks 35a-1 to 35a-3.

If an image storage instruction (in the case of the first example of the configuration shown in FIG. 1) is issued or the release switch is pressed (in the case of the second example of the configuration shown in FIG. 2) in the above-mentioned state, the mark information about the mark 35a is recorded with the image data of the endoscopic image 34a, and each "x" mark 35a can be displayed and output when the endoscopic image 34a is later displayed on the screen or output on the printer. Therefore, the operator can easily and clearly remember the examination by the mark 35a displayed with the endoscopic image, and can easily recognize the position of the biopsy, etc. from the image. Furthermore, the image can be used as is in a report, thereby reducing the laborious operation of generating a new image for the report by processing the endoscopic image.

In addition, when a plurality of marks are added to one image, it is inconvenient to use the same mark as shown in FIG. 4A.

FIG. 4B shows selecting the type of mark. In this case, the type of mark can be selected by touching a selection column 36 with the finger, etc. In the example shown in FIG. 4B, the type of mark can be selected from among "○", "×", or "△". When a desired mark is selected from the selection column 36 and the position to be marked is touched with the finger, etc., a selected mark 35b is displayed in the position. The operator can change the type of mark in the selection column 36 when the mark is added so that the mark can be used corresponding to the target to be marked. In the three marks shown in FIG. 4B, a mark 35b-1 is "○", a mark 35b-2 is "×", and mark 35b-3 is "△".

A number can also be used as a mark. FIG. 4C shows an example of a display. In FIG. 4C, the numbers 1, 2, 3, ... are displayed in the order of touch of the operator on the screen. In FIG. 4C, a mark 35c-1 of "1" is displayed in the position first specified in a marking instruction, a mark 35c-2 of "2" is displayed in the position specified second, and a mark 35c-3 of "3" is displayed in the position specified third.

In this system, the order of the marks can be easily determined by the numbers. In addition, it is not necessary for the operator to select the type of mark as shown in FIG. 4B. Furthermore, the numbers displayed in the order in which the operator touches the screen can be assigned as "1-(1), 1-(2)", etc. at an instruction of the operator, thereby efficiently assigning the numbers when, for example, a plurality of samples of tissue are removed from a polyp.

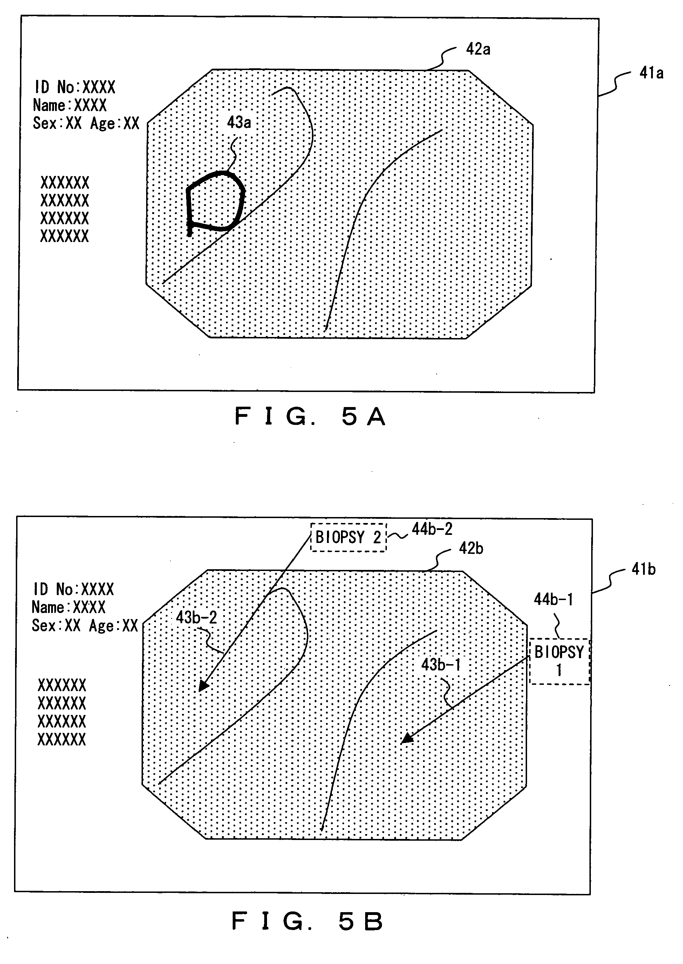

FIG. 5 shows an example of a display on the monitor 14 according to the second embodiment of the present invention.

In the second embodiment, not only a specified mark is displayed in a specified position, but also various types of information can be added to an endoscopic image. In the second embodiment, the fetched endoscopic image is recorded together with the image information indicating the contents of the added information, not with the mark information about the input mark. When data is displayed on the terminal 16, etc., the image information is combined with the endoscopic image and then displayed.

FIG. 5A shows the position of a lesion, a trace of an operation, a polyp, etc. detected in an examination enclosed by a mark.

In FIG. 5A, when the operator issues an input instruction by tracing graphics 43a with the finger, etc. by enclosing the target portion of an endoscopic image 42a, a mark generation unit 112 (112a, 112b) generates display screen data of a hand-written image at the input instruction, and a composition unit 113 (113a, 113b) combines the data with the image data from the endoscopic system 12, and displays the result on the monitor 14. The display screen data generated by the mark generation unit 112 is associated with the endoscopic image fetched by a data generation unit 114 (114a, 114b), and output to an image compression unit 111 (image compression/decompression unit 111a, image compression unit 111b). The image compression unit 111 compresses the received data, adds the header information including patient information, etc. to the data, and transmits the composite data to the server 15 for storage therein.

FIG. 5B shows an example of inputting an arrow and character information.

In FIG. 5B, the target portion of the endoscopic image 42b is specified by arrows 43b-1 and 43b-2, and the contents are expressed by character information 44b-1 and 44b-2. On a display screen 41b of the monitor 14, when the operator issues an input instruction by tracing the target portion with the finger, etc., an arrow 43 is displayed. Then, when a character is input from the input device such as a keyboard, etc. provided for the image recording device 11, the input contents are displayed as character information 44. In this state, an image storage instruction (in the case of the first example of the configuration shown in FIG. 1) is issued or the release switch is pressed (in the case of the second example of the configuration shown in FIG. 2). Then, the endoscopic image is associated with the image data for display of the arrow 43 and the character information44, and output from the data generation unit 114 to the image compression unit 111. The data is compressed and then the header information is added to the compressed data, and transmitted to the server 15 for storage therein.

Thus, when the operator calls the endoscopic image from the terminal 16 later on, the various added information can be call together. Therefore, a report, etc. can be prepared according to the information in more detail than a single image.

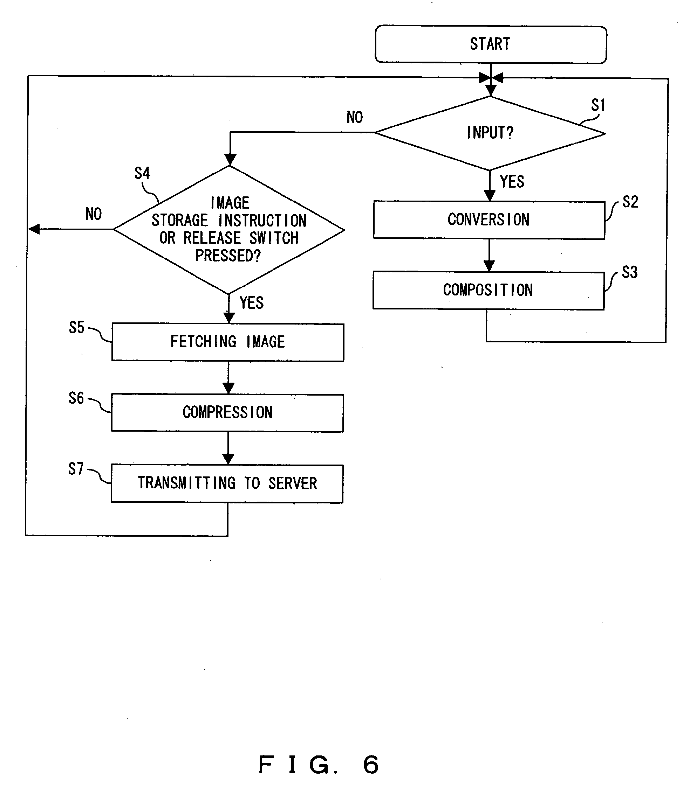

FIG. 6 is a flowchart of the process of the operations performed by an image recording device 11 (11a, 11b) when the input of a mark, etc. from the operator is added to an endoscopic image. The process shown in FIG. 6 is realized by the CPU 21 executing the firmware in the ROM 22 or the program read by the input unit 25 from a portable storage medium.

When an endoscopic examination is started, the image recording device 11 first checks in step S1 whether or not there is mark information input through a touch screen, etc. by the operator.

As a result, if there is input mark information (YES in step S1), then the mark generation unit 112 converts the contents of the input instruction into image data in step S2. In step S3, the composition unit 113 combines the image data with the endoscopic image displayed on the monitor 14, and displays the mark, etc. on the monitor 14 at the input instruction, and then control is returned to step S1.

If there is no input detected in step S1, it is determined in step S4 whether an image storage instruction has been issued (in the case of the first example of the configuration shown in FIG. 1), or the release switch has been pressed (in the case of the second example of the configuration shown in FIG. 2). If no such processes have been performed (NO in step S4), control is returned to step S1.

If it is determined in step S4 that an image storage instruction has been issued or the release switch has been pressed (YES in step S4), then an endoscopic image is fetched with the image associated with the information about the display of the mark, etc. in step S5. In step S6, the data is compressed by the image compression unit 111. Then, the header information is added to the data, transmitted to the server 15, and then control is returned to step S1. Afterwards, the above-mentioned processes are repeated until the endoscopic examination is completed.

Thus, since the instruction of the operator is combined with the endoscopic image and displayed on the monitor, the operator can input his or her own instruction while checking it on the monitor.

Information such as a mark, etc. can be added to a fetched endoscopic image and stored in the server 15. Therefore, when a fetched endoscopic image is displayed later when a report is prepared, etc., the image can be displayed together with added information.

Furthermore, when the fetched endoscopic image is displayed later on, an instruction of the operator can be displayed together. Therefore, the operator can obtain information in more detail.

The operator can use an endoscopic image as is with information added when an examination is performed. Therefore, an operation of generating a new image is not required.

In the above-mentioned examples, an operator inputs information to be added to an endoscopic image through a touch screen on the monitor 14. However, the present invention is not limited to this configuration, but any other pointing device such as a mouse, a track ball, a joy stick, etc. or any other input device such as a keyboard, a write pen, etc. can be used in inputting information.

In addition, in the above-mentioned examples, when an endoscopic image is displayed later on the terminal 16, etc., the endoscopic image is combined with the information such as mark information, etc. input by an operator, but an already composed image data can be stored in the image recording device 11 as associated with the image data of the original endoscopic image in the server 15, and these two pieces of image data can be switched and displayed when they are displayed on the terminal 16, etc.

## Claims

1. A medical image recording apparatus for recording an endoscopic image captured by an endoscope, comprising:
a generation unit generating image data to be displayed on a monitor at an instruction of an operator; and
a composition unit combining the endoscopic image captured by the endoscope with the image data, and outputting a composition result to the monitor.

2. The apparatus according to claim 1, wherein
said generation unit stops generating the image data when a predetermined time passes after generating the image data.

3. The apparatus according to claim 1 or 2, further comprising
a control unit selectively controlling whether or not said composition unit combines the endoscopic image with the image data at an instruction of an operator.

4. The apparatus according to any of claim 1 through 3, wherein
said generation unit generates first image data for a first display based on contents of a first instruction of an operator, and generates second image data for a second display on a monitor as associated with the first display based on contents of a second instruction similar to the contents of the first instruction.

5. A medical image recording apparatus for recording an endoscopic image captured by an endoscope, comprising:
a generation unit generating image data to be displayed on a monitor at an instruction of an operator; and
a recording unit recording information based on the endoscopic image captured by the endoscope as associated with information based on the image data.

6. The apparatus according to claim 5, wherein
said apparatus is connected to a server over a network, and said recording unit associates the information based on the endoscopic image captured by the endoscope with the information based on the image data, and stores the information in the server.

7. The apparatus according to any of claim 1 through 6, wherein
image data generated by said generation unit is to be used for display of a mark in a position specified by the operator.

8. The apparatus according to claim 7, further comprising
a switch unit switching a type of the mark.

9. The apparatus according to claim 7, wherein
said mark indicates an order in which the operator specifies a display position of the mark.

10. The apparatus according to claim 7, wherein
said mark includes a character string indicated by an input instruction by the operator.

11. The apparatus according to any of claim 1 through 6, wherein
said image data generated by said generation unit is used for display of a hand-written image in a position specified by the operator.

12. A method for displaying an endoscopic image by a medical image recording apparatus for recording an endoscopic image captured by an endoscope, comprising:
generating image data to be displayed on a monitor at an instruction of an operator; and
combining the endoscopic image captured by the endoscope with the image data, and displaying a composition result on the monitor.

13. A method for fetching an endoscopic image by a medical image recording apparatus for recording an endoscopic image captured by an endoscope, comprising:
generating image data to be displayed on a monitor at an instruction of an operator; and
recording information based on the endoscopic image captured by the endoscope as associated with information based on the image data.

14. A portable storage medium readable by an information processing device storing a program used to direct the information processing device, comprising when used by the information processing device for recording an endoscopic image captured by an endoscope:
generating image data to be displayed on a monitor at an instruction of an operator; and
combining the endoscopic image captured by the endoscope with the image data, and displaying a composition result on the monitor.

15. A portable storage medium readable by an information processing device storing a program used to direct the information processing device, comprising when used by the information processing device for recording an endoscopic image captured by an endoscope:
generating image data to be displayed on a monitor at an instruction of an operator; and
recording information based on the endoscopic image captured by the endoscope as associated with information based on the image data.
